# EUROPEAN PATENT APPLICATION

(11) **EP 4 616 758 A1**
(43) Date of publication of application: **17.09.2025**
(21) Application number: 24186688.8
(22) Date of filing: 04.07.2024
(51) Int. Cl.: A46B 15/00, A61B 5/00

(54) **ELECTRIC TOOTHBRUSH AND METHOD OF ANALYZING BRUSHING BEHAVIOR**

(30) Priority: 14.03.2024 US 202463565359 P
(71) Applicant: Koninklijke Philips N.V., 5656 AG Eindhoven (NL)
(72) Inventor: GUJRAL, Rashi Kumra, Eindhoven (NL); PATIL, Ravindra Balasaheb, Eindhoven (NL)
(74) Representative: Philips Intellectual Property & Standards

(57) **Abstract**

A method is provided of analyzing a tooth brushing behavior of a user with a powered toothbrush, by processing the sound of the tooth brushing session. A sensed sound signal is processed to detect when the toothbrush is on and off so that a report of (at least) brushing time is prepared.

## Description

### FIELD OF THE INVENTION

This invention relates to electric toothbrushes, and in particular it relates to providing feedback about brushing behavior.

### BACKGROUND OF THE INVENTION

Electric toothbrushes are becoming increasingly advanced, with motion sensors in the brush handles to detect brushing patterns, and apps to provide guidance and feedback about the brushing technique.

Toothbrush handles can be roughly divided into two categories; connected handles and non-connected handles. Connected handles are high-end models which come with RFID sensors and motion sensing, using a gyroscope sensor to identify which part of the mouth is being brushed. These handles can for example be paired with dedicated apps (e.g., the Philips Sonicare app) which come with many features to help users to develop better brushing habits. With the help of an app, a user can also track the number of brushing sessions remaining on their current brush handle, calculated based on brushing time. It also provides the user with a guided experience by providing a detailed summary of modes in which the brushing has been done. The app delivers better brushing outcomes by habit building and results in a consequent lower cost of oral healthcare for consumers. The app enables a personalized interactive experience to be provided to consumers.

By way of example, a connected toothbrush performs timing of brushing sessions and provides a report (e.g., weekly) of brushing behavior. The motion tracking enables timing information to be given for different tooth segments. It has also been proposed to monitor brushing forces and brush stroke directions, so that brushing forces applied to different parts of the mouth can be identified.

Non-connected toothbrush handles are low-end products which do not possess RFID sensors and cannot be connected to an app. Thus, with these handles users cannot experience guided brushing, for example guidance on duration of brushing, brushing mode, time at which pressure is applied, or a warning that the user is scrubbing.

Currently, approximately 88% of the total volume of toothbrush sales is for non-connected handles. These non-connected handles lack the connectivity to provide detailed reports about the brushing behavior. It would however be of interest to provide a connected experience even for a non-connected toothbrush handle, but without building connectivity into the handle. For example, providing a connected experience and detailed report about brushing behavior even for a non-connected toothbrush handle could give users a feeling of the advantages that a connected experience can provide provide. Thus, it would encourage users to progress to a fully connected device, which will deliver improved brushing outcomes.

### SUMMARY OF THE INVENTION

The invention is defined by the claims.

According to examples in accordance with an aspect of the invention, there is provided a method of analyzing a toothbrushing behavior of a user with a powered toothbrush, comprising:
receiving a sensed raw audio signal which captures sounds of a toothbrushing session;
processing the sensed audio signal to detect when the toothbrush is on and off; and
providing a report of brushing time.

The invention provides a system which analyzes brushing sounds to provide information about the brushing behavior of a user, even when using a non-connected toothbrush handle. As explained below, the detected sounds may for example be used to detect the duration of tooth brushing, the mode of tooth brushing (for a toothbrush with different modes of different duration) and optionally the presence of an audio pacer signal. In this way, a connected experience can be provided to consumers of non-connected devices, by adding audio sensing features (for example using AI) without adding additional hardware, thereby keeping costs low. Indeed, the audio sensing can be performed by a device remote from the toothbrush, such as a mobile phone on which a suitable app is loaded.

The audio sensing needs to be robust to detect the toothbrush sounds with different background noises. In respect of mode detection, the modes may be detected while brushing is happening or at the end of a brushing session. In respect of pacer signal sensing, a real time indicator may be provided to instruct the user to change the segment of brushing. In addition to providing a report of brushing time, a guided experience may be provided.

The audio analysis may be more involved, for example to detect additional parameters, such as the pressure applied (based on sound pitch) and the time during which pressure is applied during brushing.

The sensed audio signal is for example processed using a deep learning model. The model may be obtained by training using real brushing recordings during which measurements are made to provide ground truth information.

Processing the sensed audio signal for example comprises:
dividing the raw audio signal into chunks;
extracting spectrogram features for each raw audio signal chunk; and
labelling each chunk as brush on or brush off using the deep learning model.

Thus, time segments of audio data may be determined as brush on or brush off time periods.

Extracting spectrogram features for example comprises extracting MEL spectrogram features.

The powered toothbrush may have a plurality of brushing modes, and the method further comprises determining a brushing mode that is being used. Each different brushing mode for example has a corresponding different total brushing time. Some modes may use a pacer signal.

The method may further comprise further processing the sensed audio signal to detect if a pacer signal, which indicates to a user when they should change brushing segment, is present or not. In this way, the method may identify a pacer brushing mode.

The further processing of the sensed audio signal for example uses a deep learning model. It for example comprises:
dividing the raw audio file into chunks;
extracting spectrogram features for each raw audio chunk;
labelling each chunk as pacer signal present on pacer signal not present using the deep leaning model.

Thus, time segments of audio data may be determined as pacer signal present or not present time periods.

Extracting spectrogram features for example comprises extracting MEL spectrogram features.

The method may further comprise, if a pacer signal is detected as present, processing the sensed audio signal to determine, from the pacer signal, which tooth segment is being brushed and provide a report of brushing times per segment. The pacer signal signifies a change from one segment to another, so if the user is following a fixed segment sequence, the algorithm can track the segment being brushed in real time. The duration of brushing the segments may also indicate the brushing mode, even before the overall brushing session is completed.

The invention also provides a computer program comprising computer program code which is adapted, when said program is run on a computer, to implement the method defined above.

The invention also provides a mobile phone app comprising the computer program defined above.

The invention also provides a powered toothbrush system, comprising:
a powered toothbrush; and
the mobile phone app defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows one example of an audio processing method;
Fig. 2 shows a toothbrush and mobile phone which communicate using short range communication;
Fig. 3 shows a first example of audio sound processing;
Fig. 4 shows a second example of audio sound processing;
Fig. 5 shows how the audio pre-processing and feature extraction is performed for brushing sound analysis;
Fig. 6 shows Mel spectrogram feature extraction; and
Fig. 7 shows how mode detection is performed, at the end of a brushing session.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method of analyzing a toothbrushing behavior of a user with a powered toothbrush by processing the sound of the tooth brushing session. A sensed sound signal is processed to detect when the toothbrush is on and off so that a report of (at least) brushing time is prepared.

The invention is based on the use of audio signal processing to interpret the brushing behavior of a user. Audio signal processing is for course well known. Indeed, several machine learning and deep learning architectures have been proposed for audio classification.

One example is the UrbanSound8K algorithm, which is a public dataset. This dataset contains 8732 labeled sound extract of urban sounds often classes: car horn, dog bark, gun shot, children playing, air conditioner, drilling, vehicle engine running, jack hammer, siren, and street music. Mel Frequency Cepstral Coefficient (MFCC) feature extraction may be used (with Zero padding) to create an input for a CNN model. Feature padding rather than zero padding may instead be applied, and other features like zero-crossing rate, pitch, RMS energy, chroma along with MFCC may be used.

Another example is the ESC-50 dataset which consists of fifty classes. The dataset consists of 2000 audio samples with 5 second duration each.

Most existing pre-trained neural network models (such as Yamnet, VGG-Net etc.) are specifically designed to operate on general environmental sounds. However, the audio analysis of this invention requires detection of the particular sounds of a toothbrush for which the algorithm is designed (e.g., a Sonicare toothbrush) as well as (in preferred implementations) a specific pacer signal. Thus, pretrained models may not have sufficient sensitivity to detect the specific acoustic features to be sensed.

The invention makes use of a dedicated deep learning model, such as a CNN architecture, designed to perform well in detecting brushing sounds and preferably also pacer signal sounds. The method is able to provide a guided experience in real time.

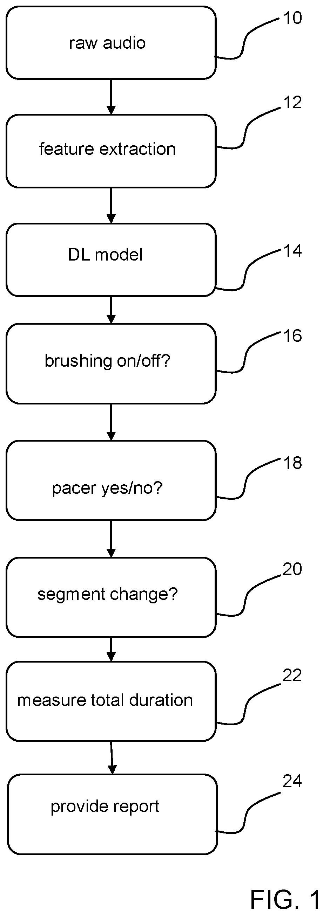

Fig. 1 shows one example of an audio processing method.

In step 10, a raw audio signal is received. It may be received by a microphone which forms part of an electric toothbrush or it may be received by a separate microphone such as the microphone of a mobile phone on which an app is loaded.

Preprocessing of the raw audio involves quantizing the data into chunks for example of duration 1 second, with an overlap for example of 0.5 seconds.

The algorithm performs feature extraction in step 12. The feature extraction for example involves extraction of time domain and frequency domain features such as a Mel spectrogram thus creating an audio feature array. A deep learning (DL) model is built using the feature array, which has an ability to detect the sound of the electric toothbrush for which the algorithm is designed (such as the Philips Sonicare power toothbrush) as well as the sound of a pacer signal which is present between the toothbrush sounds.

The sounds are detected in real time. The DL model is for example deployed in an app as an APK file which can be run on the mobile device.

The toothbrushing sound is continuous in nature, hence it is periodic and complex in nature. The toothbrush raw audio files used to build the DL model consist of toothbrushing sounds in real time of different subjects, capturing different intensities, modes and background noises. The audio length varies in dependence on the mode of the toothbrush during audio capture. For example, a clean mode duration is for example 2 minutes and a gum mode duration is for example 3 minutes 20 seconds. The files also consist of various background noises such as a door opening or closing, footsteps, a water tap, toilet flush etc. A pacer sound is used to indicate when the brushing segment is to be changed. The data is annotated whenever this pacer sound is present.

A CNN-based binary classification model is built to predict whether the brush is on or not and whether the pacer sound is present or not. The DL model is applied to the extracted features in step 14.

The model can determine if the toothbrush is on or off in step 16, if a pacer signal is present or not in step 18, and if a tooth segment change signal is present in step 20.

The total duration of a brushing session is derived in step 22, and this can be used together with the other derived information to determine the brushing mode that was used. By way of example, a toothbrush may have a cleaning mode, a whitening mode, and a and gum health mode.

A report of the brushing session is provided in step 24.

The report for example provides at least some of the information and feedback that would be provided by a connected device. Connected devices for example provide a weekly report of brushing frequency, average brush time, an indication of the suitability of the applied pressure, and an indication of whether scrubbing has been avoided. A coverage report, of how well all the different tooth segments have been covered (based on information from a gyroscope sensor), may also be provided by connected devices. Some or all of this feedback may be provided, depending on the level of audio signal analysis.

The DL algorithm needs to be robust so that it can detect the toothbrush sound and pacer sound in different background noise situations and thereby detect if the toothbrush is on or off and can detect the toothbrush mode which has been used.

The report may provide feedback about the brushing characteristics, but also may provide a guided experience. In an enhanced implementation, the audio analysis can also detect times during which pressure is applied (due to a sound change) and potentially also the level of applied pressure.

In order for the algorithm to provide reliable reporting, a toothbrushing session should be performed in one go with no interruptions in between. If audio is collected by a remote microphone, the distance between the toothbrush and sound recording device should be within a defined range, such as 60cm to 120cm. For subsegment detection, so that a sub-segment specific brushing report can be provided, the brushing session should start from a set location and follow a set sequence (e.g., starting from the upper right and then continuing in a circular way).

The method is preferably implemented for electric toothbrush handles without changing the hardware. This is possible by using the microphone of a smartphone.

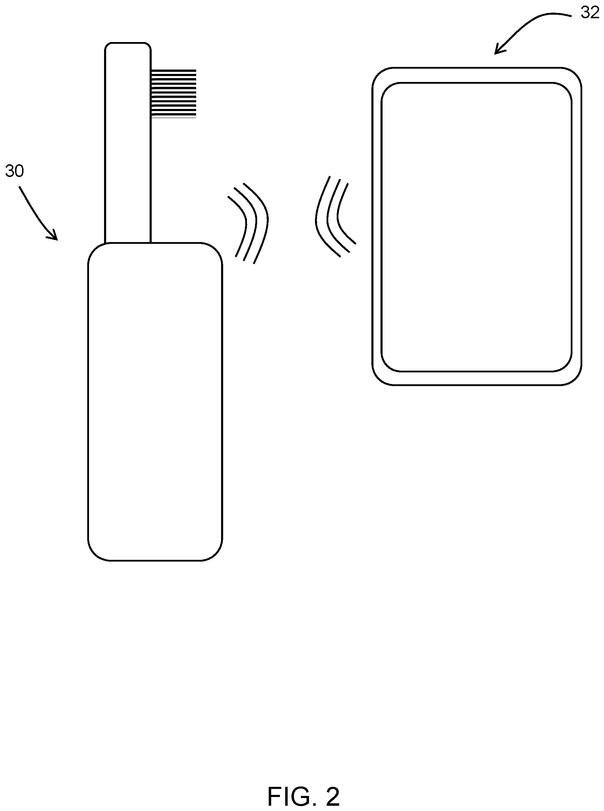

Fig. 2 shows a toothbrush 30 and mobile phone 32 which communicate using short range communication, such as over a WiFi network or using Bluetooth. A microphone of the smartphone provides the audio capture, and the algorithm is provided as an app on the smartphone. The algorithm may instead be hosted remotely and accessed by the smartphone.

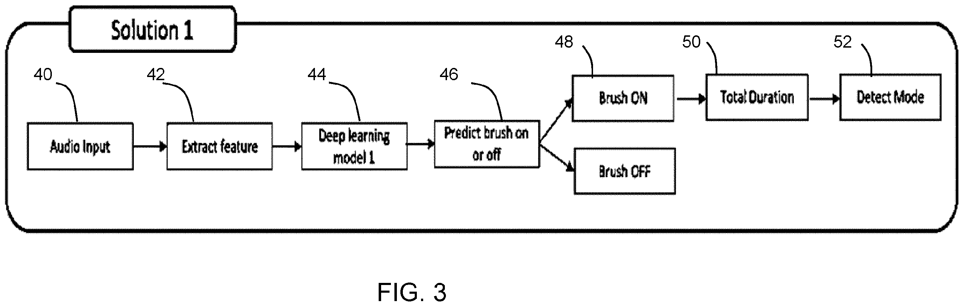

Fig. 3 shows a first example of audio sound processing.

The audio input is received in step 40. Features are extracted in step 42. The deep learning model is applied in step 44. This enables a prediction of whether the brush is on or off in step 46. If the brush is on (as determined in step 48) the total duration is obtained at the end of the brushing session in step 50 and from this the mode can be detected in step 52.

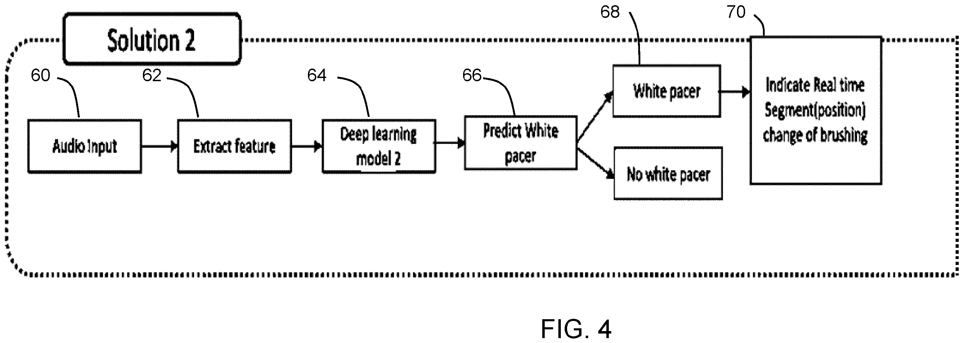

Fig. 4 shows a second example of audio sound processing.

The audio input is received in step 60. Features are extracted in step 62. The deep learning model is applied in step 64. This enables a prediction of whether a pacer signal is present. If the pacer signal is present (as determined in step 68) the real time change to the segment at which brushing is taking place is indicated as feedback in step 70.

Thus, the DL model (or models) may be used to provide brushing session information at the end of the session as well as real-time information.

A CNN-based binary classification model may be used to detect whether the brush is on or not and if a pacer is present or not. For building the model, a set of audio signals for a particular toothbrush (e.g., Sonicare Xian electric toothbrush) are collected, operating in the different modes (clean mode, white mode and gum health mode). For each mode, brushing sessions are recorded with different background noises (no noise, water tap on, and toilet flush sound).

The audio can be collected using the in-built microphone of iOS and Android devices, at a distance between 60cm and 120cm. Each brushing mode may also be performed with three intensities: low, medium and high. The audio is for example collected with a sample rate of 44100 samples/second in real time.

Annotations are then applied to extract the pacer areas from the audio signals. The extracted data consists of the start and end time of each pacer sound in the audio file. A fixed window of 1 second may for example be used.

In order to provide the annotation, the raw audio file may be listened to manually and paused to identify timing points. The area to be annotated (e.g., with a fixed window size of 1 second) is then chosen to indicate the start and end time of the brushing sound and the start and end time of the pacer sounds.

The DL algorithm is trained for brush duration and mode detection using feature extraction from the sequential audio data chunks, which have been annotated as explained above to indicate if the brush is on or off. Based on the brush on periods, the duration is calculated, and modes can then be classified by post processing based on the already set durations for the different modes (e.g., clean mode, white mode, gum mode).

The DL algorithm is trained for pacer detection also using feature extraction from the sequential audio data chunks, which have been annotated as explained above to indicate if a pacer signal is present or not. Based on pacer signals, the segment changes are identified. The audio chunks may for example have a duration of 1 second with 0.5 seconds of overlap, as the pacer is transient in nature and will have brush sounds before and after it. The overlap window avoids data loss.

After model quantization, a trained DL model for production is generated. As shown in Fig. 1, the trained DL algorithm can:
detect the pacer sounds and thereby determine when a segment change has been instructed (in real time);
determine the real time duration of the brushing so far; and
determine the brushing duration of the entire session and thereby determine the brushing mode.

The system provides real time feedback to change brushing segment, hence supplementing the pacer sound. The summary can then provide advice to indicate how well the user performed, in terms of their average brush time, relative to the desired brushing time for the particular mode they are using.

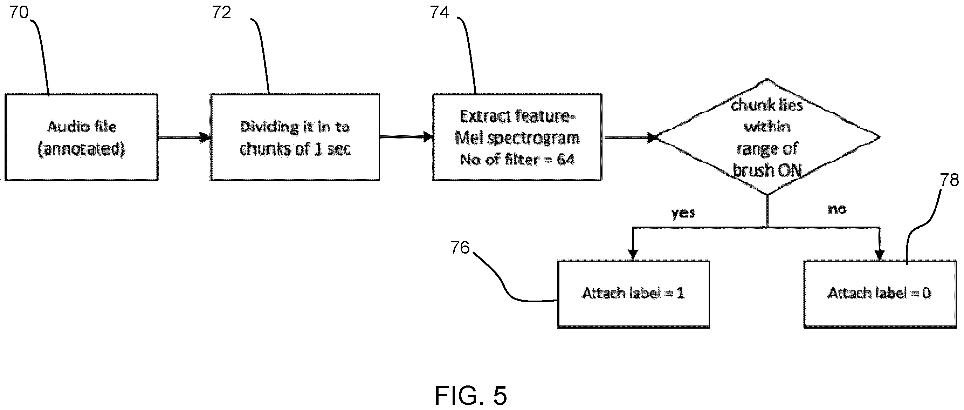

Fig. 5 shows how the audio pre-processing and feature extraction is performed for brushing sound analysis.

The audio is received in step 70. Audio pre-processing is performed by dividing the audio into 1 second chunks in step 72, extracting Mel spectrogram features in step 74 followed by labelling each chunk as brush on or not. Each frame is labelled as brush on (label 1, step 76) if the frame beginning and ending times lie in the range of any annotated power toothbrush region time, else the frame is labelled as off (label 0, step 78).

Deep learning models rarely take raw audio directly as input. The common practice is to pass an image of the audio (comprising spectrogram feature) as an input to CNN-based architectures.

A spectrogram is a visual way of representing the signal signature (loudness etc.) over time at various frequencies present in a particular waveform. The spectrogram plots Frequency (y-axis) vs. Time (x-axis) and uses different colors to indicate the amplitude of each frequency.

The Mel scale is a scale of pitches that human hearing generally perceives to be equidistant from each other. The scale is based on the comparison between pitches. Mel spectrogram features help in applications that need to model human hearing perception. Thus, Mel spectrogram data is well suited for use in this audio classification application.

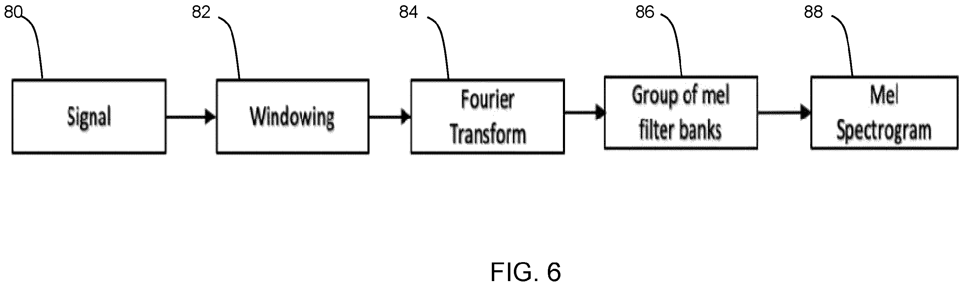

Fig. 6 shows Mel spectrogram feature extraction.

The audio signal is received in step 80. The signal is windowed in step 82, and then a Fourier Transform is applied in step 84. Groups of Mel filter banks are applied in step 86 to derive a Mel spectrogram in step 88.

By way of example, filters for 64 Mel spectrogram features may be used. This yields a very compressible representation of MFCCs, often using just 20 or 13 coefficients instead of 32-64 bands in the Mel spectrogram. The MFCC is slightly more decorrelated, which can be beneficial with linear models. With lots of data and strong classifiers like Convolutional Neural Networks, Mel-spectrogram often perform better. The Mel spectrogram is better at detecting differences in lower frequencies than higher frequencies. The output of the model is in the form of tensors.

After successfully completing the audio pre-processing and feature extraction step, the model is trained over the extracted feature tensors. The Mel spectrogram values may be used as feature extractors of the audio signals and a CNN-based deep learning approach is used for classification of the input signal. The Mel spectrogram gives good tolerance to noise and matches human sound perception.

A CNN model may be optimized for the particular application.

By way of example, the CNN model may use a rectified linear unit ReLU as the activation function layer in the middle/hidden layers and a sigmoid activation function may be used in the final detection layer.

The Adam optimizer may be used as the optimization function.

The Cost Function or Loss Function may be based on Binary Cross Entropy with logits loss.

To demonstrate the effectiveness of the approach, a CNN model as outlined above was trained with 40 audio files and tested with 14 audio files. For the Mel spectrogram a sample rate of 44.1 kbits/second was used, with n_fft=1024, hop_length=512, and n_mels=64.

This yielded the following results:

| | Training (%) | Testing (%) |
|---|---|---|
| Accuracy | 94.16 | 92.82 |
| Sensitivity | 87.43 | 86.52 |
| Specificity | 89.28 | 88.71 |

As explained above, post processing of the entire brushing session may be used to provide mode detection.

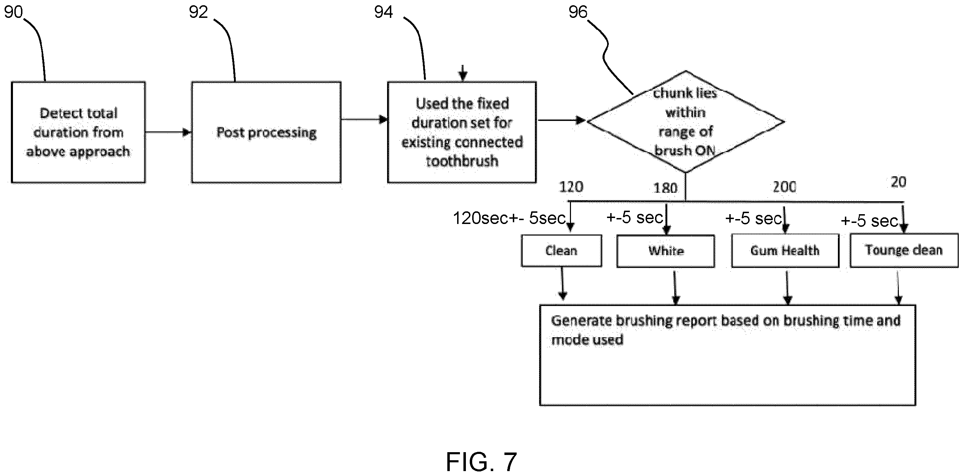

Fig. 7 shows how the mode detection is performed, at the end of a brushing session.

The total duration is derived in step 90, in the manner explained above.

Post processing is applied in step 92.

In step 94, data is received relating to the toothbrush being used, in particular the fixed duration information relating to the different modes of operation.

In step 96, chunks during which the brush was on is determined. Step 96 represents preset rules to determine the mode/type of brushing session. The previous duration is just used to classify the session into one of these segments.

The total brushing time is compared with thresholds, in this example 120 seconds, 180 seconds, 200 seconds and 20 seconds (each with a margin of +- 5 seconds) to determine the mode. The modes in this example are cleaning (120 seconds), whitening (180 seconds), gum health (200 seconds) and a tongue clean (20 seconds).

A brushing report is then generated in step 98 based on brushing time and the mode used.

The pre-processing is one of the standard major phases of machine learning inference (pre-processing, model prediction and post-processing). The pre-processing for example involves tuning data so that it is usable for the machine learning model to perform inference. In the model prediction phase a certain pre-processed input is passed to find whether it belongs to the positive or negative class in a given binary classifier. The post-processing phase reports the output of the machine learning model to conclude a meaningful result. In this case, the post-processing is the summation of all positive outputs predicted over the sequence of time in a given sample or session.

The invention may be implemented as a mobile app to provide brushing report and guided brushing to users of non-connected toothbrushes. In addition to the basic functions of pacer signal detection and on/off detection, audio analysis may also be used to detect the application of pressure (which results in a reduced sound pitch), the level of pressure applied (which influences the amount of sound pitch reduction), the presence of scrubbing (with a characteristic audio signature) and their respective duration times. This may be achieved by additional algorithm training but using the same principles as discussed above.

There may be a single DL algorithm, or multiple algorithms each for their own detection function.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method of analyzing a toothbrushing behavior of a user with a powered toothbrush, comprising:
(10) receiving a sensed raw audio signal which captures sounds of a toothbrushing session;
(14, 16) processing the sensed audio signal to detect when the toothbrush is on and off; and
(24) providing a report of brushing time.

2. The method of claim 1, comprising (14) processing the sensed audio signal using a deep learning model.

3. The method of claim 2, wherein processing the sensed audio signal comprises:
(72) dividing the raw audio signal into chunks;
(74) extracting spectrogram features for each raw audio signal chunk; and
(76, 78) labelling each chunk as brush on or brush off using the deep learning model.

4. The method of claim 3, wherein (74) extracting spectrogram features comprises extracting MEL spectrogram features.

5. The method of any one of claims 1 to 4, wherein the powered toothbrush has a plurality of brushing modes, and the method further comprises determining a brushing mode that is being used.

6. The method of claim 5, wherein each different brushing mode has a corresponding different total brushing time.

7. The method of any one of claims 1 to 6, further comprising (18) further processing the sensed audio signal to detect if a pacer signal, which indicates to a user when they should change brushing segment, is present or not.

8. The method of claim 7, wherein the further processing (18) of the sensed audio signal uses a deep learning model.

9. The method of claim 8, wherein the further processing of the sensed audio signal comprises:
dividing the raw audio file into chunks;
extracting spectrogram features for each raw audio chunk;
labelling each chunk as pacer signal present on pacer signal not present using the deep leaning model.

10. The method of claim 9, wherein extracting spectrogram features comprises extracting MEL spectrogram features.

11. The method of any one of claims 7 to 10, further comprising, if a pacer signal is detected as present, processing the sensed audio signal to determine, from the pacer signal, which tooth segment is being brushed and provide a report of brushing times per segment.

12. A computer program comprising computer program code which is adapted, when said program is run on a computer, to implement the method of any one of claims 1 to 11.

13. A mobile phone app comprising the computer program of claim 12.

14. A powered toothbrush system, comprising:
a powered toothbrush (30); and
the mobile phone app of claim 13.
